# EUROPEAN PATENT APPLICATION

(11) **EP 4 799 580 A1**
(43) Date of publication of application: **02.09.2026**
(21) Application number: 25161154.7
(22) Date of filing: 28.02.2025
(51) Int. Cl.: A61B 17/88

(54) **A BONE TRACTION ASSEMBLY FOR FRACTURE REDUCTION AND/OR REALIGNMENT**

(71) Applicant: The Provost, Fellows, Foundation Scholars, and the other members of Board, of the College of the Holy & Undiv. Trinity of Queen Elizabeth near Dublin, Dublin 2 (IE)
(72) Inventor: Boyle, Gerard, Blackrock, A94 R1X8 (IE); Henry, Cian, Glencar, F91 RH60 (IE); Soraghan, Christopher, Enfield (IE); Casey, Conall, Glasnevin, D11 C9F3 (IE); Kane, Máire, Kilmainham (IE); Ekanayake, Kumara, Dublin 8 (IE)
(74) Representative: FRKelly

(57) **Abstract**

The present invention relates to a traction assembly for the reduction and/or realignment of a bone fracture. The invention finds utility in the reduction and/or realignment of a bone such as a jawbone having a fracture by reducing and/or realigning the bone having the fracture. Also disclosed is a method of bone fracture reduction and/or realignment using the assembly.

## Description

### Field of the invention

The present invention relates to a traction assembly for the reduction and/or realignment of a bone fracture. The invention finds utility in the reduction and/or realignment of a bone such as a jawbone having a fracture by reducing and/or realigning the bone having the fracture.

### Background to the invention

A mandibular condyle fracture describes a break near the hinge joint of the jaw. Mandibular fractures are one of the most common facial bone fractures and some of the most difficult to manage, with mandible condylar fractures being the most common facial bone fracture. Causes include traumatic facial injuries such as motor vehicle accidents, violence, sports-related falls, and industrial incidents. The U-shaped mandible is the most prominent facial bone and a common site of trauma, constituting 12-56% of facial fractures. Condylar fractures account for about 29-52% of all mandibular fractures.

When a patient presents with a fracture in the condyle of the jawbone, a decision is made to either treat the fracture conservatively or with surgical intervention. Conservative management involves closed reduction and fixation, with the use of dental bars and wires maintaining the jaws in tight occlusion (upper and lower jaws held together) for a period of weeks. Intuitively, this will affect functions such as mastication (chewing), speech, etc. The closed/conservative treatment of a jaw fracture is recognised as safe, non-invasive and easy to perform but it can result in poor oral hygiene, gingivitis, facial deformity, temporomandibular disorder (TMD), and/or malocclusion.

The decision to treat the fracture surgically, for example with a metal plate across the fracture site, negates some of these complications and allows a sooner return to normal function. However, fractures of the mandibular condyle are challenging to manage surgically given the close proximity of vital structures such as the facial nerve and/or artery and limited access to the surgical site.

In recent years, there has been a trend towards increased surgical intervention (open reduction and internal fixation (ORIF)) for the treatment of mandible condylar fractures and maxillofacial surgeons have devised their own off-label methods to repair these fractures. Generally, open reduction involves surgically realigning the fracture and securing it with titanium plates. There is no standard fracture repair technique and fracture repair techniques vary from case to case with variation; such that adoption of a consistent fracture reduction technique would improve patient outcomes when compared to off-label use of ad-hoc surgical instruments for each surgery.

When a fracture is significantly displaced, applying an opposing force to reduce the fracture (to realign the jawbone together) is very challenging within the anatomical confines of the surgical site. There is a need for a device or assembly for reducing and re-align the jawbone together within the anatomical confines of the surgical site to simplify and formalise the fracture reduction process. Such a device or assembly can be a non-invasive, sterile, single-use device or assembly that can reduce overall procedural time.

Such a device or assembly would make it easier, faster, and safer to realign fractured condyles, subsequently improving the likelihood of surgical treatment being chosen over conservative management; thereby avoiding the need for wiring or elasticating the jaw shut, for what can be 6 weeks, and so improving patient experience and outcomes including: patients being able to eat solid soft food after surgery and avoid a fully liquidised diet, better chance of realigning the jaw accurately thereby facilitating normal speech, better teeth alignment, reduced pain, reduced risk of facial deformity or nerve damage, reduced risk of intraoperative haemorrhage, and minimal or invisible scar formation. Such a device or assembly could also be used to realign other fractured bones and so also find utility in other maxillofacial/orthopaedic applications.

### Summary of the invention

According to a first aspect of the present invention, there is provided a bone traction assembly comprising:
(a) a fixation element securable in a bone;
(b) a cannula having proximal and distal ends defining a lumen therebetween; and
(c) a retractable wire configured to pass through the cannula lumen and fixable to the fixation element.

Optionally, retraction of the retractable wire at the proximal end of the cannula displaces the fixation element toward the distal end of the cannula.

Optionally or additionally, retraction of the retractable wire at the proximal end of the cannula attaches the fixation element at or adjacent the distal end of the cannula. Further optionally or additionally, retraction of the retractable wire at the proximal end of the cannula reversibly attaches the fixation element at or adjacent the distal end of the cannula.

Preferably, retraction of the retractable wire at the proximal end of the cannula displaces the fixation element toward the distal end of the cannula.

Optionally, the fixation element comprises securing means for securing the fixation element in the bone.

Optionally, the securing means comprises a threaded screw.

Optionally, the fixation element comprises fixing means for fixing to the fixation element. Further optionally, the fixation element comprises fixing means for fixing to the fixation element secured in the bone.

Optionally, the fixation element comprises fixing means for fixing the retractable wire to the fixation element. Further optionally, the fixation element comprises fixing means for fixing the retractable wire to the fixation element secured in the bone.

Optionally, the fixing means comprises a screw head.

Optionally, the fixation element comprises a threaded screw having a screw head.

Preferably, the fixation element comprises a threaded screw having a screw head.

Optionally, the cannula is configured to allow passage of the retractable wire through the cannula lumen.

Optionally, the cannula is a flexible cannula. Further optionally, the cannula is a flexible tube cannula.

Optionally, the cannula is formed from a plastic material. Further optionally, the cannula is formed from a biocompatible plastic material.

Optionally, the cannula is formed from a material selected from silicone (polysiloxane), polyurethane (PU), polyethylene (PE), polyvinylchloride (PVC), polytetrafluoroethylene (PTFE), and combinations thereof.

Preferably, the cannula is formed from polyurethane (PU).

Optionally, the retractable wire is a flexible wire.

Optionally or additionally, the retractable wire is a non-elastic wire.

Optionally, the retractable wire is a monofilament wire.

Optionally, the retractable wire is a multifilament wire. Further optionally, the retractable wire is a braided multifilament wire.

Preferably, the retractable wire is a monofilament wire.

Optionally, the retractable wire is formed from a metal material. Further optionally, the retractable wire is formed from an alloy material. Still further optionally, the retractable wire is formed from an alloy of nickel and titanium.

Optionally, the retractable wire is formed from a plastic material. Further optionally, the retractable wire is formed from a biocompatible plastic material.

Optionally, the retractable wire is formed from a material selected from polyethylene (PE), polyvinylidene fluoride (PVDF), nylon, and combinations thereof.

Preferably, the retractable wire is formed from nylon.

Optionally, the retractable wire comprises capturing means for capturing the fixation element. Further optionally, the retractable wire comprises capturing means for capturing the fixation element secured in the bone.

Optionally, the retractable wire comprises capturing means for capturing the fixing means of the fixation element. Further optionally, the retractable wire comprises capturing means for capturing the fixing means of the fixation element secured in the bone.

Optionally, the capturing means comprises a loop. Further optionally, the capturing means comprises a loop integral with the retractable wire. Further optionally, the capturing means comprises a loop formed from the retractable wire.

Optionally, the retractable wire comprises respective ends defining a wire body therebetween.

Optionally, the capturing means comprises a loop integral with the wire body. Further optionally, the capturing means comprises a loop formed from the wire body.

Optionally, the retractable wire comprises respective ends defining the wire body and the capturing means therebetween. Further optionally, the retractable wire comprises respective ends defining the wire body and the loop therebetween.

Optionally, the capturing means has an annular form. Further optionally, the loop has an annular form. Optionally, the capturing means is biased toward an annular form. Further optionally, the loop is biased toward an annular form.

Optionally, the bone traction assembly further comprises a wire actuator arranged for displacement of the retractable wire. Still further optionally, the bone traction assembly further comprises a wire actuator arranged for reciprocal displacement of the retractable wire.

Optionally, the wire actuator comprises at least two sections arranged for relative reciprocal displacement. Optionally, the wire actuator comprises first and second sections arranged for relative reciprocal displacement.

Optionally, the first section of the wire actuator comprises a screw thread and the second section of the wire actuator comprises a screw thread. Further optionally, the first and second sections of the wire actuator each comprises a corresponding screw thread.

Optionally, rotation of the first section of the wire actuator relative to the second section of the wire actuator causes displacement of the first section of the wire actuator relative to the second section of the wire actuator. Further optionally, rotation of the first section of the wire actuator relative to the second section of the wire actuator causes linear displacement of the first section of the wire actuator relative to the second section of the wire actuator.

Optionally, the retractable wire is attached to the wire actuator. Further optionally, the retractable wire is secured to the wire actuator. Further optionally, the retractable wire is clamped to the wire actuator.

Optionally, the wire actuator comprises a clamp to clamp the retractable wire to the wire actuator.

Optionally, the clamp comprises at least two sections and clamping means for clamping the at least two sections together. Further optionally, the clamp comprises first and second sections and clamping means for clamping the first and second sections together.

Optionally, the section-engaging faces of the at least two sections are not smooth. Further optionally, the section-engaging faces of the first and second sections are not smooth.

Optionally, the section-engaging faces of the at least two sections comprises a plurality of solid particles or grits. Further optionally, the section-engaging faces of the first and second sections comprises a plurality of solid particles or grits.

Optionally, at least part of the retractable wire is received between the at least two sections. Further optionally, at least part of the retractable wire is received between the first and second sections. Optionally, the respective ends of the retractable wire are each received between the at least two sections. Further optionally, the respective ends of the retractable wire are each received between the first and second sections.

Optionally, the bone traction assembly further comprises deploying means for deploying the retractable wire. Further optionally, the bone traction assembly further comprises deploying means for deploying the retractable wire toward the distal end of the cannula. Still further optionally, the bone traction assembly further comprises deploying means for deploying the retractable wire beyond the distal end of the cannula.

Optionally, the deploying means comprises at least two sections arranged for relative reciprocal displacement. Optionally, the deploying means comprises first and second sections arranged for relative reciprocal displacement.

Optionally, the deploying means comprises at least two sections arranged for relative reciprocal displacement between a retracted state and a deployed state. Optionally, the wire actuator comprises first and second sections arranged for relative reciprocal displacement between a retracted state and a deployed state.

Optionally, the deploying means or the at least two sections or the first and second sections are biased toward the retracted state. Optionally, the deploying means or the at least two sections or the first and second sections comprises an elastic member, which biases the deploying means or the at least two sections or the first and second sections toward the retracted state. Optionally, the deploying means or the at least two sections or the first and second sections comprises a spring optionally a compression spring, which biases the deploying means or the at least two sections or the first and second sections toward the retracted state.

Optionally, elastic member or the spring or the compression spring is arranged between the at least two sections or the first and second sections to bias the at least two sections or the first and second sections toward the retracted state.

Optionally, the elastic member or the spring or the compression spring is integral with at least part of the deploying means or the at least two sections or the first and second sections. Further optionally, the elastic member or the spring or the compression spring is integral with at least one of the first and second sections.

Optionally, linear displacement of the first section of the deploying means relative to the second section of the deploying means causes displacement of the retractable wire. Further optionally, linear displacement of the first section of the deploying means relative to the second section of the deploying means causes displacement of the retractable wire toward the distal end of the cannula. Still further optionally, linear displacement of the first section of the deploying means relative to the second section of the deploying means causes displacement of the retractable wire beyond the distal end of the cannula.

Optionally, the retractable wire is attached to the deploying means. Further optionally, the retractable wire is secured to the deploying means.

Optionally, linear displacement of the first section of the deploying means relative to the second section of the deploying means causes displacement of the deploying means from the retracted state to the deployed state. Further optionally, displacement of the linear displacement of the first section of the deploying means relative to the second section of the deploying means causes displacement of the deploying means from the retracted state to the deployed state, such that the retractable wire is displaced beyond the distal end of the cannula.

Optionally, the bone traction assembly further comprises a retractable needle having proximal and distal ends defining a lumen therebetween.

Optionally, the needle is configured to allow passage of the cannula through the needle lumen.

Optionally, the cannula is configured to pass through the needle lumen.

Optionally, the bone traction assembly further comprises a needle actuator arranged for displacement of the retractable needle. Still further optionally, the bone traction assembly further comprises a needle actuator arranged for reciprocal displacement of the retractable needle.

Optionally, the needle actuator comprises a guide mountable to the bone traction assembly. Optionally, the needle actuator comprises a mountable guide arranged for reciprocal displacement relative to the bone traction assembly.

Optionally, the retractable needle is attached to the needle actuator. Further optionally, the retractable needle is secured to the needle actuator.

Optionally, linear displacement of the guide relative to the bone traction assembly causes linear displacement of the retractable needle relative to the bone traction assembly.

Also disclosed is a method of bone fracture reduction and/or realignment, the method comprising the steps of:
(a) providing a bone traction assembly according to a first aspect of the present invention;
(b) securing the fixation element in a bone;
(c) fixing the retractable wire to the fixation element; and
(d) retracting the retractable wire at the proximal end of the cannula.

Optionally, the retracting step thereby displaces the fixation element toward the distal end of the cannula.

Optionally or additionally, the retracting step thereby attaches the fixation element at or adjacent the distal end of the cannula. Further optionally or additionally, the retracting step thereby reversibly attaches the fixation element at or adjacent the distal end of the cannula.

Optionally, the fixing step comprises fixing the retractable wire to fixing means of the fixation element. Further optionally, the fixing step comprises fixing the retractable wire to fixing means of the fixation element secured in the bone.

Optionally, the fixing step comprises allowing passage of the retractable wire through the cannula lumen prior to fixing the retractable wire to the fixation element.

Optionally, the fixing step comprises fixing the capturing means of the retractable wire to fixing means of the fixation element. Further optionally, the fixing step comprises fixing the capturing means of the retractable wire to fixing means of the fixation element secured in the bone.

Optionally, the retracting step comprises actuating a wire actuator to displace the retractable wire.

Optionally, the retracting step comprises rotating the first section of the wire actuator comprising a screw thread relative to the second section of the wire actuator comprising a screw thread to displace the retractable wire.

Optionally, rotation of the first section of the wire actuator relative to the second section of the wire actuator causes displacement of the first section of the wire actuator relative to the second section of the wire actuator. Further optionally, rotation of the first section of the wire actuator relative to the second section of the wire actuator causes linear displacement of the first section of the wire actuator relative to the second section of the wire actuator.

Optionally, the method further comprises the step of deploying the retractable wire prior to fixing the retractable wire to the fixation element. Further optionally, the method further comprises the step of deploying the retractable wire toward the distal end of the cannula. Still further optionally, the method further comprises the step of deploying the retractable wire beyond the distal end of the cannula.

Optionally, the deploying step comprises actuating the deploying means between the retracted state and the deployed state.

Optionally, linear displacement of the first section of the deploying means relative to the second section of the deploying means causes displacement of the retractable wire. Further optionally, linear displacement of the first section of the deploying means relative to the second section of the deploying means causes displacement of the retractable wire toward the distal end of the cannula. Still further optionally, linear displacement of the first section of the deploying means relative to the second section of the deploying means causes displacement of the retractable wire beyond the distal end of the cannula.

Optionally, the method further comprises the step of displacing the retractable needle prior to fixing the retractable wire to the fixation element.

Optionally, the needle displacing step comprises actuating the needle actuator to displace the retractable needle. Still further optionally, the needle displacing step comprises actuating the needle actuator to reciprocally displace the retractable needle.

Optionally, linear displacement of the needle actuator or guide relative to the bone traction assembly causes linear displacement of the retractable needle relative to the bone traction assembly.

Optionally, the needle displacing step comprises allowing passage of the cannula through the needle.

Optionally, the method comprises the steps of:
(a) providing a bone traction assembly according to a first aspect of the present invention;
(b) securing the fixation element in a bone;
(c) displacing the retractable needle and allowing passage of the cannula through the needle;
(d) deploying the retractable wire and allowing passage of the retractable wire through the cannula;
(e) fixing the retractable wire to the fixation element; and
(f) retracting the retractable wire at the proximal end of the cannula, thereby displacing the fixation element toward the distal end of the cannula.

### Brief description of the drawings

Embodiments of the present invention will be described with reference to the accompanying drawings in which:

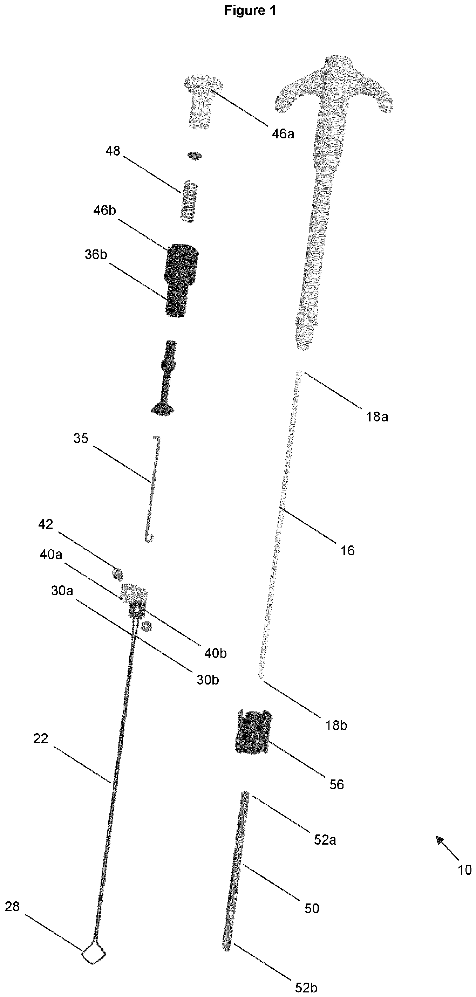

**Figure 1** is an exploded perspective view of a bone traction assembly;

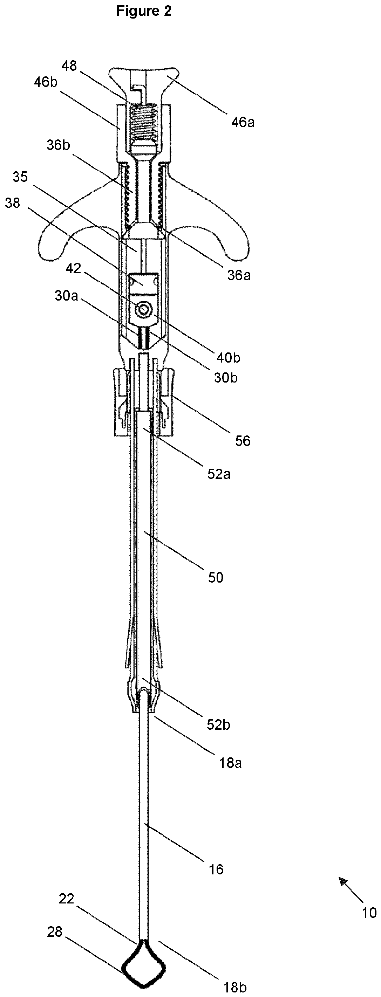

**Figure 2** is a sectional view of a bone traction assembly;

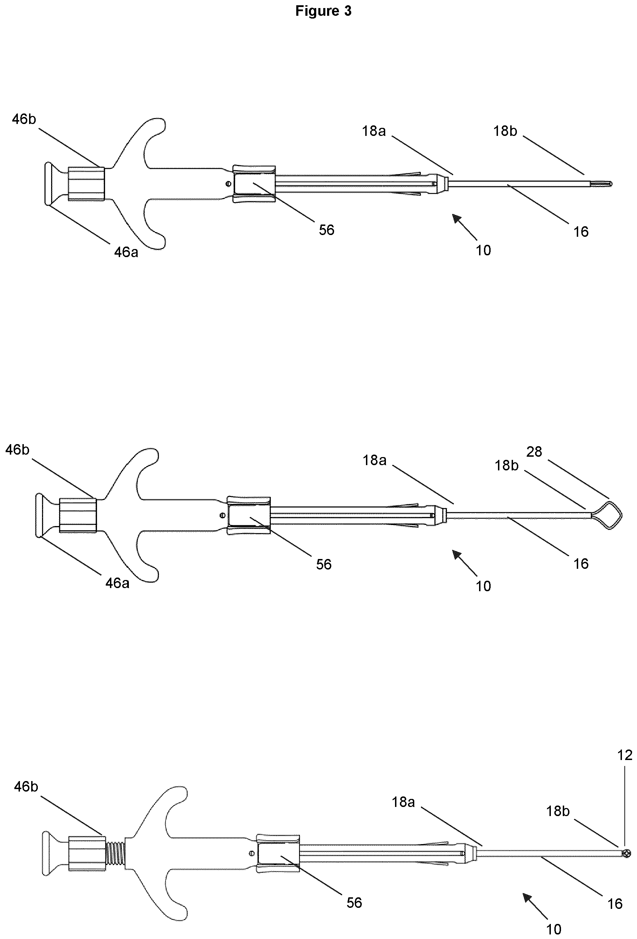

**Figure 3** is a side view of a bone traction assembly, having a retractable wire pass through a cannula (upper), having the retractable wire deployed with a capturing means or loop having an annular form (middle), and having the retractable wire fixed to a fixation element (lower);

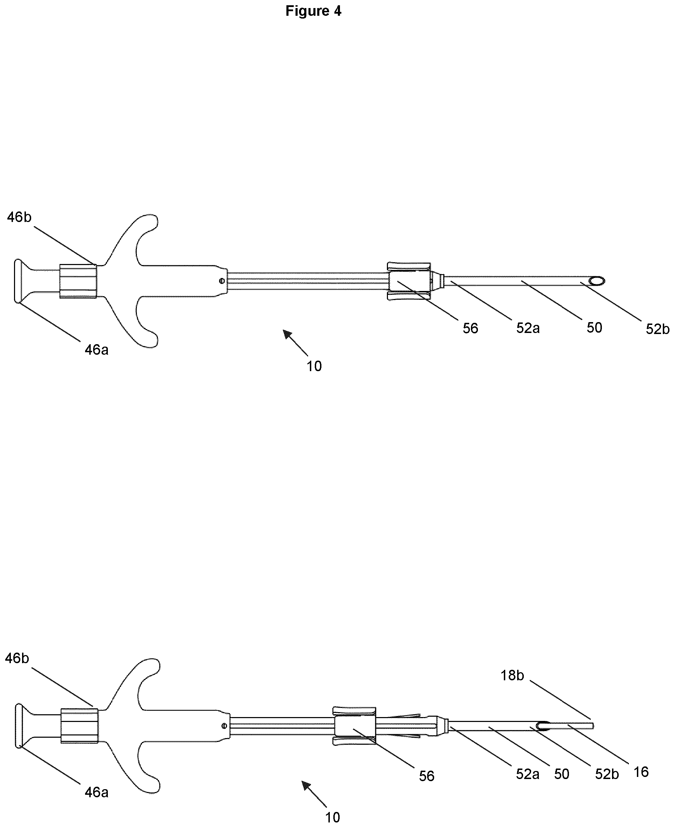

**Figure 4** is a side view of a bone traction assembly, having a retractable needle (upper), and allowing passage of a cannula through the needle (lower);

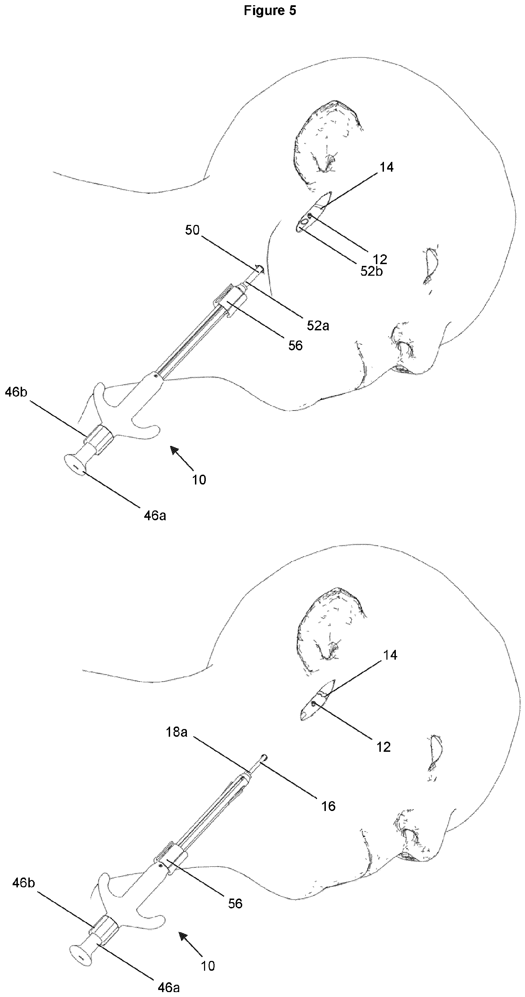

**Figure** 5 is a perspective view of a bone traction assembly having a retractable needle used to reduce and/or realign a bone fracture in a method of bone fracture reduction and/or realignment (upper), and allowing passage of a cannula through the needle (lower);and

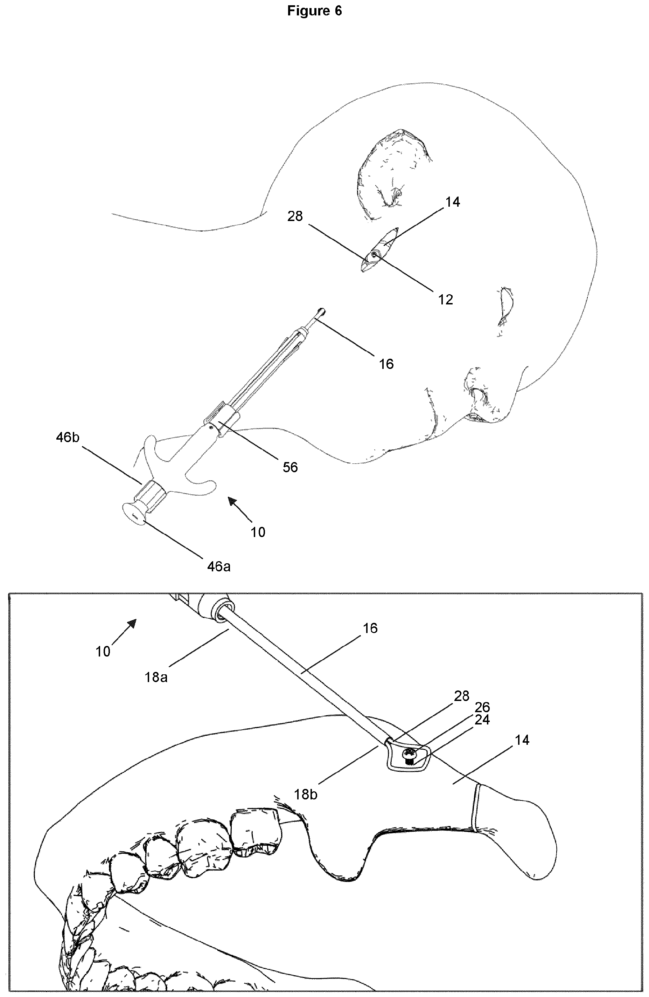

**Figure** 6 is a perspective view of a bone traction assembly having the retractable wire deployed with a capturing means or loop having an annular form used to reduce and/or realign a bone fracture in a method of bone fracture reduction and/or realignment (upper) and on an isolated/exposed bone (lower).

### Detailed description of the drawings

The present invention relates to a bone traction assembly 10. The bone traction assembly 10 comprises a fixation element 12 securable in a bone 14; a cannula 16 having proximal 18a and distal 18b ends defining a lumen 20 therebetween; and a retractable wire 22 configured to pass through the cannula lumen 20 and fixable to the fixation element 12. Retraction of the retractable wire 22 at the proximal end 18a of the cannula 16 displaces the fixation element 12 toward the distal end 18b of the cannula 16.

In a preferred embodiment, retraction of the retractable wire 22 at the proximal end 18a of the cannula 16 attaches or reversibly attaches the fixation element 12 at or adjacent the distal end 18b of the cannula 16. In such an embodiment, the fixation element 12 is displaced toward the distal end 18b of the cannula 16 and then is attached to, for example, held in place at or adjacent the distal end 18b of the cannula 16. When the cannula 16 is formed from a material selected by the person skilled in the art for the necessary strength and rigidity i.e. when the cannula 16 is rigid, the cannula can then be moved in multiple axes and, as the fixation element 12 is attached at or adjacent the distal end 18b of the cannula 16, the fixation element 12 likewise moves in multiple axes. The attachment of the fixation element 12 at or adjacent the distal end 18b of the cannula 16 can be reversed by reversing the retraction of the retractable wire 22 at the proximal end 18a of the cannula 16.

The fixation element 12 can comprise securing means 24 for securing the fixation element 12 in the bone 14. In an embodiment, the securing means 24 comprises a threaded screw that can be driven into the bone 14 and secured by and interference fit between the securing means 24 and the bone 14. The fixation element 12 can also comprise fixing means 26 for fixing (e.g. the retractable wire 22) to the fixation element 12. Generally, the fixing means 26 is for fixing the retractable wire 22 to the fixation element 12 once the fixation element 12 is secured in the bone 14. In an embodiment, the fixing means 26 comprises a screw head. In such an embodiment, wherein the securing means 24 comprises a threaded screw and the fixing means 26 comprises a screw head, the fixation element 12 can comprise a threaded screw having a screw head. The fixing means or screw head 26 is generally shaped and dimensioned to allow the retractable wire 22 to be reversibly fixed to the fixation element 12, generally once the fixation element 12 is secured in the bone 14.

The cannula 16 can be configured to allow passage of the retractable wire 22 through the cannula lumen 20. In a preferred embodiment, the cannula 16 can be a flexible cannula to facilitate displacement of the cannula 16 (e.g. bending about anatomical structures) during a method of bone fracture reduction and/or realignment. The cannula 16 generally is a flexible tube cannula having proximal 18a and distal 18b ends defining a lumen 20 therebetween. To this end, the cannula 16 is generally formed from a plastic material, and it is preferable that the cannula 16 is formed from a biocompatible plastic material, such as silicone (polysiloxane), polyurethane (PU), polyethylene (PE), polyvinylchloride (PVC), and/or polytetrafluoroethylene (PTFE). Combinations of such materials are also envisaged and can be selected by the person skilled in the art for the necessary strength, flexibility and biocompatibility.

The retractable wire 22 is generally a flexible wire. Preferably, the retractable wire is a non-elastic wire. The flexibility of the retractable wire 22 should be rigid enough to allow for passage of the retractable wire 22 through the cannula lumen 20 but flexible enough to facilitate displacement of the retractable wire 22 (e.g. bending about anatomical structures) during a method of bone fracture reduction and/or realignment. The person skilled in the art can select the retractable wire 22 or the material thereof based on the necessary strength, flexibility and biocompatibility. The retractable wire 22 can be formed from a plastic material, which is preferably a biocompatible plastic material, such as polyethylene (PE), polyvinylidene fluoride (PVDF), nylon, and/or combinations thereof. In a preferred embodiment, the retractable wire 22 is formed from nylon to provide the desired strength, flexibility and biocompatibility. The retractable wire 22 can be a monofilament wire and, to facilitate the required strength, the retractable wire 22 can be provided as a multifilament wire, and in a preferred embodiment is a braided multifilament wire.

The retractable 22 wire can comprise capturing means 28 for capturing the fixation element 12, generally once the fixation element 12 is secured in the bone 14. When the fixation element 12 comprises fixing means 26, the capturing means 28 can capture the fixing means 26 of the fixation element 12, generally when the fixation element 12 is secured in the bone 14. In a preferred embodiment, the capturing means 28 comprises a loop. In an embodiment, the capturing means 28 can be a loop that is integral with the retractable wire 22. In an embodiment, the capturing means 28 can be a loop that is itself formed from the retractable wire 22. The retractable wire 22 can have respective ends 30a,30b defining a wire body 32 therebetween. When the respective ends 30a,30b of the retractable wire 22 are brought together, a loop 28 is formed by the wire body 32, such that the loop 28 is integral with and formed from the wire body 32. It is also envisaged that the capturing means 28 can be a loop that is itself formed from the retractable wire and specifically from the wire body 32 adjacent the respective end 30a of the retractable wire 22. When the respective end 30a of the retractable wire 22 is brought adjacent or to the retractable wire 22, a loop 28 is formed by the wire body 32 between the respective end 30a and the point of contact t adjacent or to the retractable wire 22, such that the loop 28 is integral with and formed from the wire body 32 adjacent the he respective end 30a of the retractable wire 22. Alternatively, it is also possible that a separate capturing means or loop 28 is attached or secured to the retractable wire 22 or wire body 32.

In a preferred embodiment, the capturing means or loop 28 has an annular form, which can be circular or diamond or any such annular shape. The annular form of the capturing means or loop 28 facilitates capturing the fixation element 12 or fixing means 26 (once the fixation element 12 is secured in the bone 14) by facilitating the capturing means or loop 28 circumscribing the fixation element 12 or fixing means 26 by allowing the fixation element 12 or fixing means 26 to pass through the orifice defined by the annular form of the capturing means or loop 28. The shape and dimension of the capturing means or loop 28 can be planar (for example, 2-dimensional, such as elliptical, optionally circular) or non-planar (for example, 3-dimentional, such as an ellipsoid, spheroid or paraboloid, such as a hyperbolic paraboloid. However, it is envisaged that any shape and dimension of the capturing means or loop 28 that facilitates capturing the fixation element 12 or fixing means 26 can be selected an implemented by the person skilled in the art. The capturing means or loop 28 can be biased toward the annular form. For example, by thermosetting or strengthening the retractable wire 22 at the site of the capturing means or loop 28, an annular form can be set, such that the retractable wire 22 retains the necessary strength and flexibility, but also retains the annular form of the capturing means or loop 28. The retractable wire 22 retaining the necessary flexibility allows the retractable wire 22 to pass through the cannula lumen 20 while still allowing an annular form of the capturing means or loop 28 - and, by having the capturing means or loop 28 biased toward the annular form, deployment of the retractable wire 22 beyond the distal end 18b of the cannula 16 allows the annular shape of the capturing means or loop 28 to be maintained after deployment of the retractable wire 22 beyond the distal end 18b of the cannula 16, and even for the annular form to be shaped an dimensioned to be greater than the cannula lumen 20.

In an embodiment, the bone traction assembly 10 can further comprise a wire actuator 34 arranged for displacement, preferably reciprocal displacement, of the retractable wire 22. The wire actuator 34 can comprise at least two sections arranged for relative reciprocal displacement. In a preferred embodiment, the wire actuator 34 comprises first 36a and second 36b sections arranged for relative reciprocal displacement. The first section 36a of the wire actuator 34 can comprise a screw thread and the second section 36b of the wire actuator can comprise a screw thread. The first and second sections 36a,36b each preferably comprise a corresponding screw thread, such that the screw thread of the first section 36a engages with the screw thread of the second section 36b. For example, the inner surface of the first section 36a of the wire actuator 34 can comprise a screw thread and the outer surface of the second section 36b of the wire actuator can comprise a screw thread whereby the screw thread of the first section 36a engages with the screw thread of the second section 36b such that the second section 36b can co-locate with and move relative to the first section 36a. In such an embodiment, rotation of the first section 36a of the wire actuator 34 relative to the second section 36b of the wire actuator causes displacement of the first section 36a of the wire actuator 34 relative to the second section 36b of the wire actuator 34, generally linear displacement in the longitudinal direction of the bone traction assembly 10. Such an arrangement provides the necessary torque required to consistently and continually reciprocally displace the retractable wire 22, in particular to consistently and continually retract the retractable wire 22 once fixed to the fixation element 12 secured in the bone 14. However, it is envisaged that any arrangement of the wire actuator 34 that allows displacement, preferably reciprocal displacement, of the retractable wire 22 can be implemented in the bone traction assembly 10. The retractable wire 22 is attached to the wire actuator 34. The retractable wire 22 can be adhered, including over-moulded, or tied to the wire actuator 34 but, preferably, the retractable wire 22 is secured, fixed, or bonded to the wire actuator 34.

In a preferred embodiment, the retractable wire 22 is clamped to the wire actuator 34. In such an embodiment, the wire actuator 34 comprises a clamp 38 to clamp the retractable wire 22 to the wire actuator 34. The clamp 38 can comprise at least two sections, such as first 40a and second 40b sections, and clamping means 42 for clamping the at least two sections together. The section-engaging faces of the first 40a and second 40b sections are preferably not smooth to provide sufficient frictional force between the first 40a and second 40b sections to maintain the first 40a and second 40b sections together and to retain the retractable wire 22 between the first 40a and second 40b sections. The section-engaging faces of the at first 40a and second 40b sections can have a plurality of solid particles or grits (e.g. sandpaper), which can be regular, irregular or patterned, but it is envisaged that any surface treatment that will provide the necessary frictional force to retain the retractable wire 22 between the first 40a and second 40b sections can be selected an implemented by the person skilled in the art. The retractable wire 22 is received and clamped between the first 40a and second 40b sections and the clamping means 42 are used to clamp the first 40a and second 40b sections together. The clamping means 42 can be a nut and corresponding bolt that pass through and or are engaged from opposing sides of the clamp 38, but any means that will provide the necessary frictional force to the clamp 28 to retain the retractable wire 22 between the first 40a and second 40b sections can be selected an implemented by the person skilled in the art. In the embodiment wherein the respective ends 30a,30b of the retractable wire 22 are brought together to form a loop 28 by the wire body 32, such that the loop 28 is integral with and formed from the wire body 32, the respective ends 30a,30b of the retractable wire 22 can each received between the first 40a and second 40b sections.

The wire actuator 34 can further comprise connecting means 35. In an embodiment, the clamp 38 can further comprise connecting means 35. The connecting means 35 can connect the wire actuator 34 and the retractable wire 22. The connecting means 35 can connect the clamp 38 and the retractable wire 22. The connecting means 35 can be a shaft, optionally a rigid shaft, further optionally a solid, rigid shaft. The connecting means 35 can generally be formed from a metal material but could also be formed from a plastic material. Such materials can be selected by the person skilled in the art for the necessary strength and rigidity.

In an embodiment, the bone traction assembly 10 can further comprise deploying means 44 for deploying the retractable wire 22. The deploying means 44 can deploy the retractable wire 22 toward the distal end 18b of the cannula 16, and can deploy the retractable wire 22 beyond the distal end 18b of the cannula 16. The deploying means 44 can comprise first 46a and second 46b sections arranged for relative reciprocal displacement, generally between a retracted state and a deployed state. The first 46a and second 46b sections are biased toward the retracted state. For example, an elastic member 48, which can be a spring such as a compression spring, biases the first 46a and second 46b sections toward the retracted state. In this embodiment, the elastic member or the spring 48 is arranged between the first 46a and second 46b sections to bias the first 46a and second 46b sections toward the retracted state, but any means that will provide the necessary biasing force to the deploying means 44 to deploy the retractable wire 22 can be selected an implemented by the person skilled in the art. Pushing on the first section 46a causes linear displacement of the first section 46a relative to the second section 46b against the bias of the elastic member or the spring 48, thereby displacing the deploying means 44 from the retracted state to the deployed state. Since the retractable wire 22 is attached or secured to the deploying means 44 in particular the first section 46a, pushing on the first section 46a causes displacement of the retractable wire 22 toward the distal end 18b of the cannula 16 and then beyond the distal end 18b of the cannula 16. The elastic member or the spring or the compression spring 48 can generally be formed from a metal material but could also be formed from a plastic material. Such materials can be selected by the person skilled in the art for the necessary strength, flexibility and compression. In an embodiment, the elastic member or the spring or the compression spring 48 can be integral with at least part of the deploying means 44 or the at least two sections or the first 46a and second 46b sections. In such an embodiment, the elastic member or the spring or the compression spring 48 can generally be formed from the same material as the at least part of the deploying means 44 or the at least two sections or the first 46a and second 46b sections; for example formed from a plastic material.

The bone traction assembly 10 can further comprise a retractable needle 50 having proximal 52a and distal 52b ends defining a lumen 54 therebetween. The needle 50 can be configured shaped or dimensioned to allow passage of the cannula 16 through the needle lumen 54, whereby the cannula 16 can also be shaped or dimensioned to pass through the needle lumen 54. In such an embodiment comprising a retractable needle 50, the bone traction assembly 10 can also further comprise a needle actuator 56 arranged for displacement, preferably reciprocal displacement, of the retractable needle 50. The needle actuator 56 is arranged for displacement or reciprocal displacement and can be secured or locked in a distal and/or proximal position relative to the path of displacement. The needle actuator 56 can comprise a guide mountable to the bone traction assembly 10. The mountable guide 56 can be arranged for reciprocal displacement relative to the bone traction assembly 10, for example, by circumscribing at least part of the bone traction assembly 10 and so being arranged for slidable reciprocal displacement relative to the bone traction assembly 10. The retractable needle 50 is attached or secured to the mountable guide 56. For example, the mountable guide 56 can pass through an orifice on the surface of the bone traction assembly 10 and be attached or secured to the retractable needle 50. The orifice can be a longitudinal orifice that permits linear displacement of the guide 56 relative to the bone traction assembly 10, thereby causing linear displacement of the retractable needle 50 relative to the bone traction assembly 10. The needle actuator 56 can be arranged for displacement or reciprocal displacement by deforming or squeezing the needle actuator 56. Optionally, the needle actuator 56 can be arranged for displacement or reciprocal displacement by deforming or squeezing the mountable guide 56. Such an embodiment comprising a retractable needle 50 avoids the risk of accidental penetration of the skin by retractable needle 50.

To use the bone traction assembly 10 to reduce and/or realign a bone fracture, a method of bone fracture reduction and/or realignment can comprise the steps of providing a bone traction assembly 10 according to a first aspect of the present invention; securing the fixation element 12 in a bone 14; fixing the retractable wire 22 to the fixation element 12; and retracting the retractable wire 22 at the proximal end 18a of the cannula 16, thereby displacing the fixation element 12 toward the distal end 18b of the cannula 16.

In an embodiment comprising a retractable needle 50, once the fixation element 12 is secured in the bone 14, the bone traction assembly 10 can be directed to the site of the bone 14 using the retractable needle 50. The method can then comprise the step of displacing the retractable needle 50 by actuating the needle actuator 56 to displace, preferably reciprocally displace, the retractable needle 50. Linear displacement of the needle actuator or guide 56 relative to the bone traction assembly 10 causes linear displacement of the retractable needle 50 relative to the bone traction assembly 10, thereby retracting the needle and exposing the cannula 16. The needle displacing step can also additionally comprise allowing passage of the cannula 16 through the needle lumen 54.

The fixing step generally comprises allowing passage of the retractable wire 22 through the cannula lumen 20. The method can then comprise the step of deploying the retractable wire 22 toward the distal end 18b of the cannula 16 and then beyond the distal end 18b of the cannula 16 by actuating the deploying means between the retracted state and the deployed state. Linear displacement of the first section 46a relative to the second section 46b causes the displacement of the retractable wire 22. The retractable wire 22 retains the necessary flexibility to pass through the cannula lumen 20 while still allowing an annular form of the capturing means or loop 28 and, by having the capturing means or loop 28 biased toward the annular form, the annular shape of the capturing means or loop 28 is maintained after deployment of the retractable wire 22 beyond the distal end 18b of the cannula 16. The annular form of the capturing means or loop 28 facilitates capturing the fixation element 12 or fixing means 26.

The fixing step can comprise fixing the retractable wire 22 to the fixing means or screw head 26 of the fixation element 12. Preferably, the fixing step comprises fixing the capturing means or loop 28 of the retractable wire 28 to the fixing means or screw head 26 of the fixation element 12.

The retracting step comprises actuating the wire actuator 34 to displace the retractable wire 22. In an embodiment, the retracting step comprises rotating the first section 36a comprising a screw thread relative to the second section 36b of the wire actuator comprising a corresponding screw thread to displace or retract the retractable wire 22. Rotation of the first section 36a relative to the second section 36b causes linear displacement of the first section 36a relative to the second section 36b generally linear displacement in the longitudinal direction of the bone traction assembly 10.

The method can therefore provide bone fracture reduction and/or realignment by providing a bone traction assembly according to a first aspect of the present invention; securing the fixation element in a bone; displacing the retractable needle and allowing passage of the cannula through the needle; deploying the retractable wire and allowing passage of the retractable wire through the cannula; fixing the retractable wire to the fixation element; and retracting the retractable wire at the proximal end of the cannula, thereby displacing the fixation element toward the distal end of the cannula.

## Claims

1. A bone traction assembly comprising:
(a) a fixation element securable in a bone;
(b) a cannula having proximal and distal ends defining a lumen therebetween; and
(c) a retractable wire configured to pass through the cannula lumen and fixable to the fixation element.

2. A bone traction assembly according to claim 1, wherein retraction of the retractable wire at the proximal end of the cannula displaces the fixation element toward the distal end of the cannula.

3. A bone traction assembly according to claim 1 or 2, wherein retraction of the retractable wire at the proximal end of the cannula reversibly attaches the fixation element at or adjacent the distal end of the cannula.

4. A bone traction assembly according to any one of claims 1-3, wherein the fixation element comprises a threaded screw having a screw head.

5. A bone traction assembly according to any one of claims 1-4, wherein the retractable wire is a monofilament wire formed from nylon.

6. A bone traction assembly according to any one of claims 1-5, wherein the retractable wire comprises capturing means comprising a loop formed from the retractable wire for capturing the fixation element.

7. A bone traction assembly according to claim 6, wherein the capturing means has an annular form and is biased toward the annular form.

8. A bone traction assembly according to any one of claims 1-7, further comprising a wire actuator comprising first and second sections arranged for relative reciprocal displacement.

9. A bone traction assembly according to claim 8, wherein the first and second sections of the wire actuator each comprises a corresponding screw thread, whereby rotation of the first section of the wire actuator relative to the second section of the wire actuator causes linear displacement of the first section of the wire actuator relative to the second section of the wire actuator.

10. A bone traction assembly according to claim 8 or 9, wherein the wire actuator comprises a clamp comprising first and second sections and clamping means for clamping the first and second sections together, and wherein at least part of the retractable wire is received between the at least two sections.

11. A bone traction assembly according to claim 10, wherein the section-engaging faces of the first and second sections comprises a plurality of solid particles or grits.

12. A bone traction assembly according to any one of claims 1-11, further comprising deploying means comprising first and second sections arranged for relative reciprocal displacement between a retracted state and a deployed state, wherein the deploying means are biased toward the retracted state, whereby linear displacement of the first section of the deploying means relative to the second section of the deploying means causes displacement of the deploying means from the retracted state to the deployed state, such that the retractable wire is displaced beyond a distal end of the cannula.

13. A bone traction assembly according to any one of claims 1-12, further comprising a retractable needle having proximal and distal ends defining a lumen therebetween to allow passage of the cannula through the needle lumen.

14. A bone traction assembly according to claim 13, further comprising a needle actuator comprising a guide mountable to the bone traction assembly arranged for reciprocal displacement relative to the bone traction assembly and attached to the retractable needle, whereby linear displacement of the guide relative to the bone traction assembly causes linear displacement of the retractable needle relative to the bone traction assembly.
